(19) Europäisches Patentamt
European Patent Office
Office européen des brevets

(11) EP 3 916 002 A1

(12) **EUROPEAN PATENT APPLICATION**
published in accordance with Art. 153(4) EPC

(43) Date of publication:
**01.12.2021 Bulletin 2021/48**

(21) Application number: **20745803.5**

(22) Date of filing: **17.01.2020**

(51) Int Cl.:
*C07K 1/22* (2006.01) *C07K 16/00* (2006.01)
*C12M 1/00* (2006.01) *B01J 20/06* (2006.01)

(86) International application number:
**PCT/JP2020/001499**

(87) International publication number:
**WO 2020/153254 (30.07.2020 Gazette 2020/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.01.2019 JP 2019010358**

(71) Applicants:
- **NGK SPARK PLUG CO., LTD.**
**Mizuho-ku**
**Nagoya-shi,**
**Aichi 467-8525 (JP)**
- **NATIONAL INSTITUTE OF ADVANCED INDUSTRIAL SCIENCE AND TECHNOLOGY**
**Chiyoda-ku**
**Tokyo 100-8921 (JP)**

(72) Inventors:
- **KATO, Katsuya**
**Nagoya-shi, Aichi 463-8560 (JP)**
- **NAGATA, Fukue**
**Nagoya-shi, Aichi 463-8560 (JP)**
- **KASAHARA, Shinjiro**
**Nagoya-shi, Aichi 467-8525 (JP)**
- **HIROBE, Yuki**
**Nagoya-shi, Aichi 467-8525 (JP)**
- **OTSUKA, Jun**
**Nagoya-shi, Aichi 467-8525 (JP)**

(74) Representative: **Zimmermann & Partner Patentanwälte mbB**
**Postfach 330 920**
**80069 München (DE)**

(54) **IMMUNOGLOBULIN PURIFICATION METHOD AND IMMUNOGLOBULIN PURIFICATION DEVICE, AND IMMUNOGLOBULIN PRODUCTION METHOD AND IMMUNOGLOBULIN PRODUCTION DEVICE**

(57) To provide an immunoglobulin purification method which achieves a high immunoglobulin recovery percentage without causing loss of the antibody nature of an immunoglobulin. The immunoglobulin purification method includes an adsorption step and a desorption step. The adsorption step involves adsorption of an immunoglobulin onto porous zirconia particles in a neutral buffer. The desorption step involves desorption of the immunoglobulin adsorbed on the porous zirconia particles from the porous zirconia particles by means of a neutral desorption liquid.

EP 3 916 002 A1

## Description

### Technical Field

The present invention relates to an immunoglobulin purification method, to an immunoglobulin purification device, to an immunoglobulin production method, and to an immunoglobulin production device.

### Background Art

Immunoglobulins are useful as therapeutic drugs and extracorporeal diagnostic agents. It is predicted that demand for immunoglobulins will continue to increase in the future. Use of an immunoglobulin for such an application requires development of a technique for purifying the immunoglobulin at a high level. In general, immunoglobulin is purified through affinity chromatography using protein A (see, for example, Patent Document 1). Protein A exhibits binding specificity to IgG and is separated from the pellicle of Staphylococcus aureus. Protein A binds specifically to immunoglobulins derived from various animals, and the amount of bound immunoglobulin per unit protein is large. Thus, affinity chromatography using a protein A-immobilized carrier is used in an antibody purification process on an industrial scale.

### Prior Art Documents

### Patent Documents

Patent Document 1: Japanese Patent Application Laid-Open (kokai) No. 2017-47365

### Summary of the Invention

### Problems to be Solved by the Invention

However, affinity chromatography employing protein A uses an acidic solution having a pH of 4 or less for elution of an adsorbed immunoglobulin, and thus may cause a change in the high-order structure of the antibody, leading to association and aggregation of the antibody.

Also, an immunoglobulin aggregate is formed at a cell culture stage, and affinity chromatography employing protein A encounters difficulty in removing an impurity containing such an immunoglobulin polymer (aggregate).

In order to solve the aforementioned problems, ion-exchange chromatography or hydrophobic interaction chromatography is generally performed after purification through affinity chromatography employing protein A.

However, conventional ion-exchange chromatography has a narrow range of conditions under which only an immunoglobulin monomer can be separated, and thus causes a problem of low immunoglobulin recovery percentage. In addition, hydrophobic interaction chromatography causes a problem of high immunoglobulin purification cost, due to low immunoglobulin recovery percentage and a long period of time required for immunoglobulin recovery.

The present invention has been conceived under such circumstances, and thus an object of the invention is to provide an immunoglobulin purification method, an immunoglobulin purification device, an immunoglobulin production method, and an immunoglobulin production device, each of which achieves a high immunoglobulin recovery percentage without causing loss of the antibody nature of an immunoglobulin. The present invention can be implemented in the following modes.

### Means for Solving the Problems

[1] An immunoglobulin purification method characterized by comprising:

an adsorption step of adsorbing an immunoglobulin onto porous zirconia particles in a neutral buffer; and
a desorption step of desorbing the immunoglobulin adsorbed on the porous zirconia particles from the porous zirconia particles by means of a neutral desorption liquid.

[2] An immunoglobulin purification method as described in [1], wherein the desorption liquid is a phosphate buffer.
[3] An immunoglobulin purification method as described in [1] or [2], wherein the desorption liquid contains at least one species selected from the group consisting of a compound represented by the following formula (1):

[F1]

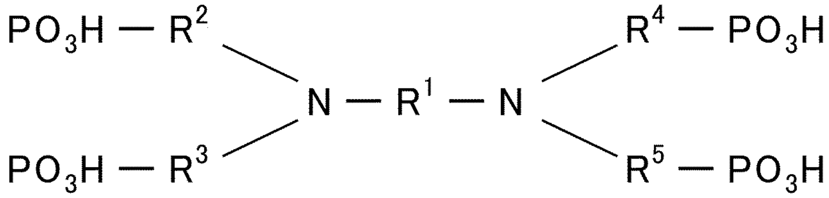

(1)

(wherein $R^1$ represents a C1 to C10 alkylene group; and each of $R^2$ to $R^5$, which may be identical to or different from one another, represents a C1 to C10 alkylene group) and a salt thereof.

[4] An immunoglobulin purification device characterized by comprising:

an adsorption unit for adsorbing an immunoglobulin onto porous zirconia particles in a neutral buffer; and
a desorption unit for desorbing the immunoglobulin adsorbed on the porous zirconia particles from the porous zirconia particles by means of a neutral desorption liquid.

[5] An immunoglobulin purification device as described in [4], wherein the desorption liquid is a phosphate buffer.
[6] An immunoglobulin purification device as described in [4] or [5], wherein the desorption liquid contains at least one species selected from the group consisting of a compound represented by formula (1):

[F2]

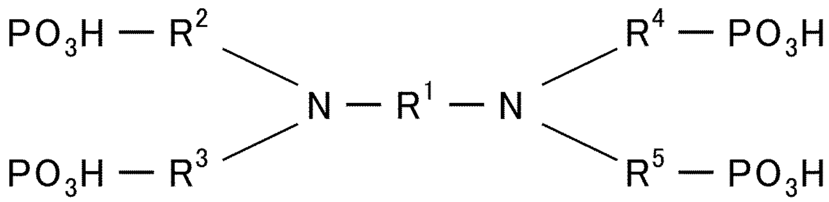

(1)

(wherein $R^1$ represents a C1 to C10 alkylene group; and each of $R^2$ to $R^5$, which may be identical to or different from one another, represents a C1 to C10 alkylene group) and a salt thereof.

[7] An immunoglobulin production method characterized by comprising:

an adsorption step of adsorbing an immunoglobulin onto porous zirconia particles in a neutral buffer; and
a desorption step of desorbing the immunoglobulin adsorbed on the porous zirconia particles from the porous zirconia particles by means of a neutral desorption liquid.

[8] An immunoglobulin production method as described in [7], wherein the desorption liquid is a phosphate buffer.
[9] An immunoglobulin production method as described in [7] or [8], wherein the desorption liquid contains at least one species selected from the group consisting of a compound represented by formula (1):

[F3]

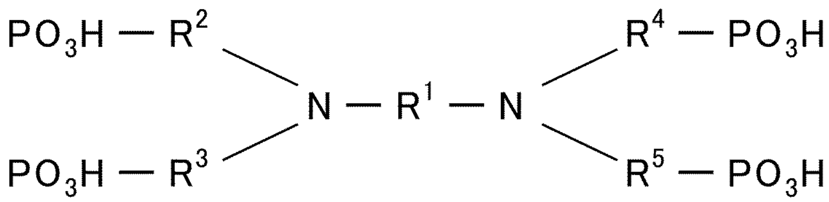

(1)

(wherein $R^1$ represents a C1 to C10 alkylene group; and each of $R^2$ to $R^5$, which may be identical to or different from one another, represents a C1 to C10 alkylene group) and a salt thereof.

[10] An immunoglobulin production device characterized by comprising:

an adsorption unit for adsorbing an immunoglobulin onto porous zirconia particles in a neutral buffer; and
a desorption unit for desorbing the immunoglobulin adsorbed on the porous zirconia particles from the porous zirconia particles by means of a neutral desorption liquid.

[11] An immunoglobulin production device as described in [10], wherein the desorption liquid is a phosphate buffer.
[12] An immunoglobulin production device as described in [10] or [11], wherein the desorption liquid contains at least one species selected from the group consisting of a compound represented by formula (1):

[F4]

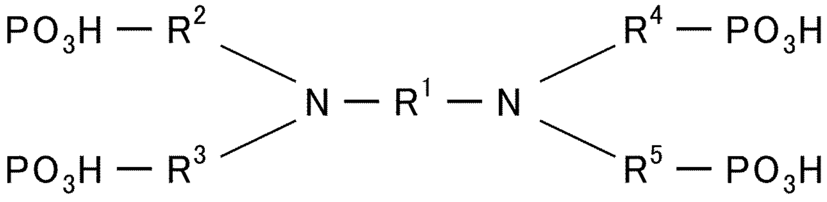

(1)

(wherein $R^1$ represents a C1 to C10 alkylene group; and each of $R^2$ to $R^5$, which may be identical to or different from one another, represents a C1 to C10 alkylene group) and a salt thereof.

Effects of the Invention

According to the immunoglobulin purification method of the present invention, an immunoglobulin is adsorbed onto porous zirconia particles in a neutral buffer, and the immunoglobulin is desorbed from the porous zirconia particles by means of a neutral desorption liquid. Thus, the immunoglobulin does not lose its antibody nature. The immunoglobulin purification method of the present invention achieves a high immunoglobulin recovery percentage.
When the immunoglobulin purification method of the present invention uses a phosphate buffer as a desorption liquid, the method achieves a high immunoglobulin recovery percentage.
In the immunoglobulin purification method of the present invention, when the desorption liquid contains at least one species selected from the group consisting of a compound represented by formula (1) and a salt of the compound, the method achieves a high immunoglobulin recovery percentage.
According to the immunoglobulin purification device of the present invention, an immunoglobulin is adsorbed onto porous zirconia particles in a neutral buffer, and the immunoglobulin is desorbed from the porous zirconia particles by means of a neutral desorption liquid. Thus, the immunoglobulin does not lose its antibody nature. The immunoglobulin purification device of the present invention achieves a high immunoglobulin recovery percentage.
When the immunoglobulin purification device of the present invention uses a phosphate buffer as a desorption liquid, the device achieves a high immunoglobulin recovery percentage.
In the immunoglobulin purification device of the present invention, when the desorption liquid contains at least one species selected from the group consisting of a compound represented by formula (1) and a salt of the compound, the device achieves a high immunoglobulin recovery percentage.
According to the immunoglobulin production method of the present invention, an immunoglobulin is adsorbed onto porous zirconia particles in a neutral buffer, and the immunoglobulin is desorbed from the porous zirconia particles by means of a neutral desorption liquid. Thus, the immunoglobulin does not lose its antibody nature. The immunoglobulin production method of the present invention achieves high immunoglobulin production efficiency.
When the immunoglobulin production method of the present invention uses a phosphate buffer as a desorption liquid, the method achieves high immunoglobulin production efficiency.
In the immunoglobulin production method of the present invention, when the desorption liquid contains at least one species selected from the group consisting of a compound represented by formula (1) and a salt of the compound, the method achieves high immunoglobulin production efficiency.
According to the immunoglobulin production device of the present invention, an immunoglobulin is adsorbed onto porous zirconia particles in a neutral buffer, and the immunoglobulin is desorbed from the porous zirconia particles by means of a neutral desorption liquid. Thus, the immunoglobulin does not lose its antibody nature. The immunoglobulin production device of the present invention achieves high immunoglobulin production efficiency.
When the immunoglobulin production device of the present invention uses a phosphate buffer as a desorption liquid, the device achieves high immunoglobulin production efficiency.
In the immunoglobulin production device of the present invention, when the desorption liquid contains at least one species selected from the group consisting of a compound represented by formula (1) and a salt of the compound,

the device achieves high immunoglobulin production efficiency.

Brief Description of the Drawings

Fig. 1 is a schematic representation of a conceivable bonding structure of ethylenediaminetetramethylenephosphonic acid (EDTPA).
Fig. 2 is a block diagram showing an immunoglobulin purification device.
Fig. 3 is a block diagram showing an immunoglobulin production device.

Modes for Carrying Out the Invention

The present invention will next be described in detail. As used herein, unless otherwise specified, the numerical value range expressed by "(value) to (value)" includes the lower limit and the upper limit of the range. For example, the expression "10 to 20" includes both a lower limit of "10" and an upper limit of "20." That is, "10 to 20" is equivalent to "10 or more and 20 or less."

1. Immunoglobulin purification method

The immunoglobulin purification method of the present invention includes an adsorption step and a desorption step.

(1) Adsorption step

The adsorption step involves adsorption of an immunoglobulin onto porous zirconia particles in a neutral buffer.

(1.1) Neutral buffer

The neutral buffer is preferably a buffer having a pH of 6.1 or more and 8.0 or less, more preferably a buffer having a pH of 6.5 or more and 7.5 or less, still more preferably a buffer having a pH of 6.6 or more and 7.4 or less. The buffer contains a buffering agent, and the buffering agent may be appropriately selected depending on the target pH from among buffering agents generally used by those skilled in the art. Examples of the buffering agent include phosphate salts, acetate salts, carbonate salts, and Tris (tris(hydroxymethyl)aminomethane). No particular limitation is imposed on the neutral buffer concentration, but the concentration is preferably 0.1 mM or more and 200 mM or less, more preferably 1 mM or more and 100 mM or less, still more preferably 5 mM or more and 20 mM or less.
The neutral buffer is particularly preferably phosphate buffered saline (PBS) from the viewpoint of maintaining the antibody nature of an immunoglobulin.
No particular limitation is imposed on the temperature of the neutral buffer, and it is generally used at a temperature of 4°C or higher and 50°C or lower.

(1.2) Immunoglobulin

The immunoglobulin may be IgG, IgA, IgM, IgD, or IgE. IgG, IgD, or IgE is in the form of monomer, IgA is in the form of monomer or dimer, and IgM is in the form of pentamer or hexamer. Each of these immunoglobulins has a size of about 10 nm to about 30 nm.

(1.3) Porous zirconia particles

No particular limitation is imposed on the porous zirconia particles used in the present invention, but the below-described porous zirconia particles are preferred. Specifically, the porous zirconia particles used in the present invention preferably have D50, D90, and a total pore volume falling within the ranges described below.
The porous zirconia particles may be in the form of individually separate particles, or may be in the form of an aggregate composed of a plurality of aggregated particles.

(1.3.1) D50, D90, and total pore volume

The porous zirconia particles have a pore diameter D50, at which a ratio of a cumulative pore volume to a total

pore volume is 50%, of 3.20 nm or more and 6.50 nm or less, and a pore diameter D90, at which a ratio of a cumulative pore volume to a total pore volume is 90%, of 10.50 nm or more and 100.00 or nm or less, as determined in a pore diameter distribution measured through a BET method. The pore diameter D50 is preferably 3.35 nm or more and 6.30 nm or less, more preferably 3.50 nm or more and 5.00 nm or less. The pore diameter D90 is preferably 10.80 nm or more and 50.00 nm or less, more preferably 11.00 nm or more and 30.00 nm or less.

The total pore volume of the porous zirconia particles is greater than 0.10 $cm^3/g$. The total pore volume is preferably greater than 0.15 $cm^3/g$, more preferably greater than 0.30 $cm^3/g$. No particular limitation is imposed on the upper limit of the total pore volume, and it is generally 10 $cm^3/g$.

When D50, D90, and the total pore volume satisfy the above conditions, the selectivity to the immunoglobulin to be adsorbed is enhanced. Notably, through adjusting D90 to be 100.00 nm or less, selective adsorption of a free (non-aggregated) immunoglobulin is facilitated. A free immunoglobulin has a size of about 10 nm to about 30 m, while an aggregate of immunoglobulin has a size of about 100 nm. Thus, D90 regulated to 100.00 nm or less avoids adsorption of an aggregate of immunoglobulin and facilitates selective adsorption of a free immunoglobulin.

Notably, the pore diameter distribution and pore volume may be determined by means of, for example, a porosity analyzer (Micromeritics, automated surface area/porosity analyzer (TriStar II, product of Shimadzu Corporation)).

The calculation method will now be described.

Firstly, a method for calculating D50 is described. By use of data of a pore diameter distribution, two points A and B, which are respectively present above and below the line representing cumulative pore volume 50% and in the closest vicinity of the 50% line, are chosen. The cumulative pore volume (X(%)) and the pore diameter (Y(nm)) of each of the points A and B are read off. Specifically, point A (Xa(%), Ya(nm)) and point B (Xb(%), Yb(nm)) are read (wherein Xa>Xb, Ya>Yb). From these values, D50 is calculated by the following calculation formula (1):

$$D50 = \log(Xb) + ((\log(Xa) - \log(Xb))*[(50 - (Yb))/((Ya) - (Yb))] \quad \cdots \text{ calculation formula (1).}$$

In a similar manner, D90 is determined. Specifically, by use of the data of the pore diameter distribution, two points C and D, which are respectively present above and below the line representing cumulative pore volume 90% and in the closest vicinity of the 90% line, are chosen. The cumulative pore volume (X(%)) and the pore diameter (Y(nm)) of each of the points C and D are read off. More specifically, point C (Xc(%), Yc(nm)) and point D (Xd(%), Yd(nm)) are read (wherein Xc>Xd, Yc>Yd). From these values, D90 is calculated by the following calculation formula (2):

$$D90 = \log(Xd) + ((\log(Xc) - \log(Xd))*[(90 - (Yd))/((Yc) - (Yd))] \quad \cdots \text{ calculation formula (2).}$$

(1.3.2) Particle diameter

No particular limitation is imposed on the particle diameter of the porous zirconia particles. However, the primary particles generally have a particle diameter of 10 nm to 100 nm, preferably 10 nm to 50 nm, more preferably 10 nm to 30 nm. Through aggregation of such primary particles, secondary particles having a particle size of 50 nm to 1,000 nm are formed. When the primary particle diameter satisfies the above conditions, the specific surface area of the porous zirconia particles considerably increases, whereby the amount of adsorbed immunoglobulin tends to increase.

(1.3.3) Chelating agent-bound porous zirconia particles

Onto the surfaces of the porous zirconia particles, a chelating agent may be bound. By virtue of the chelating agent supported on the zirconia particles, immunoglobulin adsorption selectivity can be further enhanced.

No particular limitation is imposed on the chelating agent, and the chelating agent is preferably at least one species selected from the group consisting of a compound represented by the following formula (2), ethylenediaminetetraacetic acid (EDTA), diethylenetriaminepentaacetic acid (DETPA), diethylenetriaminepentamethylenephosphonic acid (DETPPA), and salts thereof. Examples of preferred salts include alkali metal (e.g., sodium) salts.

[F5]

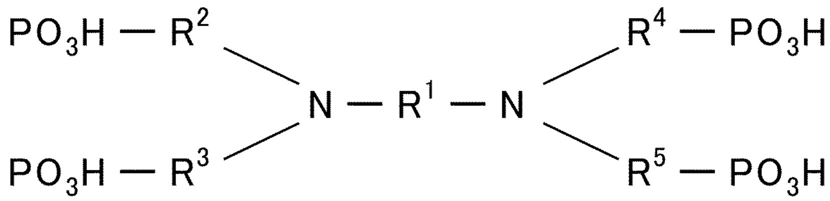

(2)

(In formula (2), $R^1$ represents a C1 to C10 alkylene group. Each of $R^2$ to $R^5$, which may be identical to or different from one another, represents a C1 to C10 alkylene group.)

Examples of the C1 to C10 alkylene group of $R^1$ in formula (2) include a methylene group, an ethylene group, a trimethylene group, a tetramethylene group, a hexamethylene group, and an isobutylene group.

Examples of the C1 to C10 alkylene group of $R^2$ to $R^5$ include a methylene group, an ethylene group, a trimethylene group, a tetramethylene group, a hexamethylene group, and an isobutylene group.

From the viewpoint of enhancing selectivity to immunoglobulin, the chelating agent is preferably a compound represented by formula (2). Among compounds represented by formula (2), ethylenediaminetetramethylenephosphonic acid (i.e., N,N,N',N'-ethylenediaminetetrakis(methylenephosphonic acid)) (EDTPA) is particularly preferred.

No particular limitation is imposed on the amount of the bound chelating agent. From the viewpoint of enhancing selectivity to immunoglobulin, the amount of bound chelating agent is preferably 0.01 μg to 10 μg per 1 mg of zirconia, more preferably 0.02 μg to 5 μg, still more preferably 0.05 μg to 3 μg.

Notably, the amount of the bound chelating agent may be calculated by a reduction in weight as determined through TG-DTA (thermogravimetry-differential thermal analysis).

No precise bonding mode of the chelating agent has been elucidated. However, the mode is assumed such that a ligand derived from the chelating agent is bound to zirconium atoms. In the case where the chelating agent is ethylenediaminetetramethylenephosphonic acid, the bonding mode is assumed to be represented by the structure shown in Fig. 1.

(1.3.4) Porous zirconia particle production method

No particular limitation is imposed on the method for producing the porous zirconia particles. The porous zirconia particles may be produced through, for example, the following method. Specifically, a zirconium oxychloride ($ZrOCl_2 \cdot 8H_2O$) solution is prepared from zircon as a raw material. Subsequently, $Zr(OH)_4$ microparticles are formed through hydrolysis of the solution. The microparticles are fired, to thereby yield porous zirconia particles.

(1.4) Relationship between amount of porous zirconia particles and amount of neutral buffer

No particular limitation is imposed on the relationship between the amount of the porous zirconia particles and the amount of the neutral buffer in the adsorption step. For example, the neutral buffer may be used in an amount of 10 μL or more and 300 μL or less with respect to 1 mg of the porous zirconia particles.

(2) Desorption step

The desorption step involves desorption (elution) of the immunoglobulin adsorbed on the porous zirconia particles from the porous zirconia particles by means of a neutral desorption liquid.

(2.1) Neutral desorption liquid

The neutral desorption liquid is preferably a desorption liquid having a pH of 6.0 or more and 8.0 or less, more preferably a desorption liquid having a pH of 6.5 or more and 7.5 or less, still more preferably a desorption liquid having a pH of 6.8 or more and 7.2 or less.

The desorption liquid is preferably a phosphate buffer. The phosphate buffer is preferably, for example, phosphate buffered saline (PBS).

No particular limitation is imposed on the desorption liquid concentration, and the concentration is appropriately determined depending on the type of the desorption liquid.

When, for example, phosphate buffered saline (PBS) is used, the concentration is preferably 50 mM or more and 200 mM or less, more preferably 60 mM or more and 150 mM or less, still more preferably 70 mM or more and 130

mM or less, from the viewpoint of enhancing the immunoglobulin recovery percentage. When phosphate buffered saline (PBS) is used as the neutral buffer in the adsorption step, and phosphate buffered saline (PBS) is used as the desorption liquid in the desorption step, these two liquids preferably satisfy the following concentration relationship from the viewpoint of enhancing the immunoglobulin recovery percentage. Specifically, the phosphate buffered saline (PBS) used in the desorption step preferably has a concentration higher than that of the phosphate buffered saline (PBS) used in the adsorption step.

The desorption liquid may contain at least one species selected from the group consisting of a compound represented by formula (1) and a salt thereof.

[F6]

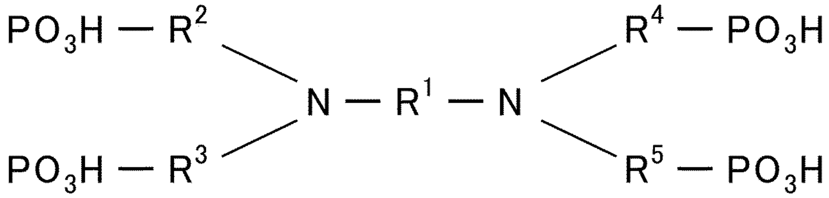

(1)

(In formula (1), $R^1$ represents a C1 to C10 alkylene group. Each of $R^2$ to $R^5$, which may be identical to or different from one another, represents a C1 to C10 alkylene group.)

Examples of the C1 to C10 alkylene group of $R^1$ in formula (1) include a methylene group, an ethylene group, trimethylene group, a tetramethylene group, a hexamethylene group, and an isobutylene group.

Examples of the C1 to C10 alkylene group of $R^2$ to $R^5$ include a methylene group, an ethylene group, a trimethylene group, a tetramethylene group, a hexamethylene group, and an isobutylene group.

From the viewpoint of enhancing the immunoglobulin recovery percentage, at least one species selected from the group consisting of a compound represented by formula (1) and a salt of the compound is preferably at least one species selected from among ethylenediaminetetramethylenephosphonic acid (EDTPA; N,N,N',N'-ethylenediaminetetrakis(methylenephosphonic acid)) and a salt thereof.

From the viewpoint of enhancing the immunoglobulin recovery percentage, the desorption liquid contains at least one species selected from the group consisting of a compound represented by formula (1) and a salt of the compound at a concentration (total concentration in the case of two or more species) of preferably 0.1 mM or more and 10 mM or less, more preferably 0.5 mM or more and 8 mM or less, still more preferably 2 mM or more and 6 mM or less.

Notably, no particular limitation is imposed on the temperature of the desorption liquid, and it is generally used at a temperature of 4°C or higher and 50°C or lower.

(2.2) Relationship between amount of porous zirconia particles and amount of desorption liquid

No particular limitation is imposed on the relationship between the amount of the porous zirconia particles and the amount of the desorption liquid in the desorption step. For example, the desorption liquid can be used in an amount of 10 μL or more and 300 μL or less with respect to 1 mg of the porous zirconia particles.

2. Immunoglobulin purification device

An immunoglobulin purification device 1 according to the present invention includes an absorption unit and a desorption unit. Fig. 2 shows an example of the immunoglobulin purification device 1, including an absorption/desorption unit 3 serving as both an absorption unit and a desorption unit. The absorption/desorption unit 3 has, for example, a tubular portion having two openings, and the tubular portion is charged with porous zirconia particles 4. The immunoglobulin purification device 1 also includes an adsorption liquid container 5 for holding a neutral buffer (adsorption liquid), a desorption liquid container 7 for holding a neutral desorption liquid, and a recovery container 9 for recovering a liquid discharged from the absorption/desorption unit 3. In the adsorption step (adsorption process), a neutral buffer (adsorption liquid) containing an immunoglobulin is supplied from the adsorption liquid container 5 to the absorption/desorption unit 3 by means of a pump, whereby the immunoglobulin is adsorbed onto the porous zirconia particles 4. In the desorption step (desorption process), a neutral desorption liquid is supplied from the desorption liquid container 7 to the absorption/desorption unit 3 by means of the pump, whereby the immunoglobulin adsorbed on the porous zirconia particles 4 is desorbed (eluted). The switching between the adsorption liquid and the desorption liquid is performed by means of a switching unit 11.

The terms "neutral buffer," "immunoglobulin," "porous zirconia particles," and "neutral desorption liquid" used

in relation to the immunoglobulin purification device 1 are the same as those described in relation to the section "1. Immunoglobulin purification method" above.

3. Immunoglobulin production method

The immunoglobulin production method of the present invention includes an adsorption step and a desorption step.
The "adsorption step" and "desorption step" of the immunoglobulin production method are the same as those described above in the section "1. Immunoglobulin purification method" above.
The immunoglobulin production method of the present invention can produce an immunoglobulin having a purity higher than that of an immunoglobulin (raw immunoglobulin) used in the adsorption step. The raw immunoglobulin is obtained through any known method. For example, the raw immunoglobulin may be separated and collected from blood or tissue fluid, may be prepared through biosynthesis or chemosynthesis, or may be prepared through a culture process. The immunoglobulin production method of the present invention may include a step of obtaining a raw immunoglobulin.

4. Immunoglobulin production device

An immunoglobulin production device 21 according to the present invention includes an absorption unit and a desorption unit. Fig. 3 shows an example of the immunoglobulin production device 21, which includes an absorption/desorption unit 23 serving as both an absorption unit and a desorption unit. The absorption/desorption unit 23 has, for example, a tubular portion having two openings, and the tubular portion is charged with porous zirconia particles 24. The immunoglobulin purification device 21 also includes an adsorption liquid container 25 for holding a neutral buffer (adsorption liquid), a desorption liquid container 27 for holding a neutral desorption liquid, and a recovery container 29 for recovering a liquid discharged from the absorption/desorption unit 23. In the adsorption step (adsorption process), a neutral buffer (adsorption liquid) containing an immunoglobulin is supplied from the adsorption liquid container 25 to the absorption/desorption unit 23 by means of a pump, whereby the immunoglobulin is adsorbed onto the porous zirconia particles 24. In the desorption step (desorption process), a neutral desorption liquid is supplied from the desorption liquid container 27 to the absorption/desorption unit 23 by means of the pump, whereby the immunoglobulin adsorbed on the porous zirconia particles 24 is desorbed (eluted). The switching between the adsorption liquid and the desorption liquid is performed by means of a switching unit 31.
The terms "neutral buffer," "immunoglobulin," "porous zirconia particles," and "neutral desorption liquid" used in relation to the immunoglobulin production device 21 are the same as those described in relation to the section "1. Immunoglobulin purification method" above.
The immunoglobulin production device 21 of the present invention can produce an immunoglobulin having a purity higher than that of an immunoglobulin (raw immunoglobulin) used in the adsorption unit. The raw immunoglobulin is obtained through any known method. For example, the raw immunoglobulin may be separated and collected from blood or tissue fluid, may be prepared through biosynthesis or chemosynthesis, or may be prepared through a culture process. The immunoglobulin production device 21 of the present invention may include a unit for obtaining a raw immunoglobulin.

Examples

The present invention will next be described in more detail.

1. Experiment A

(1) Porous zirconia particles

Porous zirconia particle products as listed in Table 1 were used.
In Test Examples 1 to 9, PBS (phosphate buffered saline) which is neutral buffer was used as an adsorption liquid. In Test Example 10, a HEPES (2-[4-(2-hydroxyethyl)-1-piperazinyl]ethanesulfonic acid) solution which is acidic solution was used as an adsorption liquid.

Table 1

| Test Ex. | Particle type | Carrier amount (mg) | Absorption liquid | | | Absorption percentage (%) | | |
|---|---|---|---|---|---|---|---|---|
| | | | Component | Concentration | pH | IgG | HAS | Trf |
| 1 | PCS140(SD) | 3 | PBS | 10mM | 7. 0 | 92. 7 | 13. 4 | 13.3 |
| 2 | PCS140(SD)-P (0.00125M) | 3 | PBS | 10mM | 7. 0 | 88. 2 | 5. 1 | 11.8 |
| 3 | PCS140(SD)-P (0.0025M) | 3 | PBS | 10mM | 7. 0 | 85. 9 | 7. 7 | 4.0 |
| 4 | PCS140(SD)-P (0.00125M) | 3 | PBS | 10mM | 7. 0 | 88. 2 | 5. 1 | 11.8 |
| 5 | PCS140(SD)-P (0.00125M) | 3 | PBS | 50mM | 7.0 | 47. 3 | 0.8 | 0. 3 |
| 6 | PCS140(SD)-P (0.00125M) | 3 | PBS | 100mM | 7.0 | 21. 9 | 0. 8 | 0. 0 |
| 7 | PCS140(SD)-P (0.00125M) | 3 | PBS | 10mM | 6.5 | 85. 8 | 15.2 | 31. 7 |
| 8 | PCS140(SD)-P (0.00125M) | 3 | PBS | 10mM | 7. 0 | 88. 2 | 5.1 | 11.8 |
| 9 | PCS140(SD)-P (0.00125M) | 3 | PBS | 10mM | 7. 5 | 69. 2 | 2. 5 | 0.0 |
| 10 | PCS140(SD) | 3 | HEPES | 10mM | 6.0 | 31. 7 | 72. 7 | 60.2 |

In Table 1, "PCS140(SD)" is a porous zirconia particle product of Nippon Denko Co., Ltd.

In Table 1, "PCS140(SD)-P (0.00125M)" is EDTPA-bound porous zirconia particles prepared by treating a porous zirconia particle product of Nippon Denko Co., Ltd. (PCS140(SD), raw material) with 0.00125M EDTPA solution.

In Table 1, "PCS140(SD)-P (0.0025M)" is EDTPA-bound porous zirconia particles prepared by treating a porous zirconia particle product of Nippon Denko Co., Ltd. (PCS140(SD), raw material) with 0.0025M EDTPA solution. These abbreviations shall apply to Tables 2 and 3 below.

"PCS140(SD)-P (0.00125M)" was prepared through the following procedure. Specifically, porous zirconia particles (PCS140(SD)) were preliminarily dried at 100°C for two hours under degassing. To the thus-dried product (250 mg) was added 0.00125M EDTPA solution (10 mL), and the resultant mixture was degassed for 15 minutes and stirred and/or shaken for 17 hours, followed by further refluxing the mixture for 4 hours. The resultant product was then washed with pure water and freeze-dried, to thereby yield PCS140(SD)-P (0.00125M).

"PCS140(SD)-P (0.0025M)" was prepared in the same manner as employed in the preparation of "PCS140(SD)-P (0.00125M)." Specifically, the procedure of preparing "PCS140(SD)-P (0.00125M)" was repeated, except that "0.00125M EDTPA solution" was replaced with "0.0025M EDTPA solution, to thereby prepare "PCS140(SD)-P (0.0025M)."

The amount of bound EDTPA was calculated from a weight loss as determined through TG-DTA (thermogravimetry-differential thermal analysis). Specifically, EDTPA-bound porous zirconia particles (about 10 mg) were weighed, and a change in weight of the sample was measured in a temperature range of ambient temperature to 1,000°C by means of a thermogravimetry-differential thermal analyzer (TG-DTA) (Thermo plus TG8120, product of Rigaku). From the weight loss in the range of 200°C to 600°C, the porous zirconia particles were found to have EDTPA bound onto the surfaces thereof in an amount of 0.06 μg to 2.20 μg per 1 mg of the particles.

(2) Evaluation of adsorption

<Test Examples 1 to 9>

Each type of porous zirconia particles shown in Table 1 was evaluated in terms of protein adsorbability.

As described below, each type of porous zirconia particles was tested with respect to three proteins; i.e., IgG, HAS (albumin from human serum), and Trf (transferrin human).

Specifically, an adsorption liquid (500 μL) was added to a Spitz tube, and porous zirconia particles (3 mg) were added to the liquid. After completion of sufficient dispersion of the porous zirconia particles, a protein liquid (500 μg/500 μL) (500 μL) was added to the dispersion, and the resultant mixture was stirred overnight at 4°C under shielding from light.
The Spitz tube was subjected to centrifugation at 12,000 rpm for 10 minutes, to thereby precipitate the porous zirconia particles. The mass of non-immobilized protein remaining in the supernatant was determined by means of a microplate reader (Infinite F200PRO, product of TECAN) using a protein assay stain (product of BIO-RAD). The difference between the initial protein mass and the mass of non-adsorbed protein was employed as the mass of adsorbed protein. The ratio of the mass of adsorbed protein to the initial protein mass was calculated as an adsorption percentage (%).

<Test Example 10>

Test Example 10 was performed in the same manner as employed in Test Examples 1 to 9, except that the amount of protein added was changed; specifically, a protein liquid (750 μg/500 μL) was added in an amount of 500 μL.

(3) Test results

Test results are shown in Table 1.

(3.1) Effect of type of adsorption liquid

The effect of the type of an adsorption liquid on protein adsorbability is assessed.
Firstly, the results of Test Examples 1 to 3 and 10 are compared with one another. The IgG adsorption percentage is higher in Test Examples 1 to 3 than in Test Example 10. The results indicate that the use of a neutral buffer as an adsorption liquid increases the IgG adsorption percentage.
Next, IgG selectivity is assessed from the results of Test Examples 1 and 10. In Test Example 10, HAS and Trf are adsorbed in addition to IgG. In Test Example 10, the IgG adsorption percentage is 31.7%, the HAS adsorption percentage is 72.7%, and the Trf adsorption percentage is 60.2%. As shown in Test Example 10, the HAS or Trf adsorption percentage is higher than IgG adsorption percentage; i.e., the selectivity to IgG adsorption is low.
In Test Example 1, HAS and Trf are adsorbed in addition to IgG, but the adsorption HAS or Trf percentage is lower than IgG adsorption percentage; i.e., the selectivity to IgG adsorption is high.
These results indicate that the use of a neutral buffer as an adsorption liquid can increase the selectivity to IgG adsorption and achieve efficient IgG adsorption. Since IgG recovery is performed through desorption of the adsorbed IgG, efficient adsorption of IgG by using a neutral buffer as an adsorption liquid leads to an increase in IgG recovery percentage.

(3.2) Effect of bound chelating agent

The effect of binding a chelating agent to porous zirconia particles on protein adsorbability is assessed.
The results of Test Example 1 (wherein EDTPA-unbound porous zirconia particles were used) are compared with those of Test Examples 2 and 3 (wherein EDTPA-bound porous zirconia particles were used). In Test Examples 2 and 3, the ratio of the HAS or Trf adsorption percentage to IgG adsorption percentage is low (i.e., the selectivity to IgG adsorption is high) as compared with the case of Test Example 1. Thus, when the chelating agent is bound to the porous zirconia particles, IgG selectivity is further improved, and the IgG recovery percentage is further enhanced.

(3.3) Effect of adsorption liquid concentration

The effect of adsorption liquid concentration on protein adsorbability is assessed.
Specifically, the results of Test Examples 4 to 6 are compared with one another. The comparison shows that the IgG adsorption percentage is the highest in Test Example 4 wherein the adsorption liquid concentration is 10 mM.
The results indicate that when the adsorption liquid concentration is adjusted to 5 mM or more and 20 mM or less, the IgG adsorption percentage is increased, whereby the IgG recovery percentage is enhanced.

(3.4) Effect of pH of adsorption liquid

The effect of the pH of an adsorption liquid on protein adsorbability is assessed.
Specifically, the results of Test Examples 7 to 9 are compared with one another. The comparison shows that the IgG adsorption percentage is the highest in Test Example 8 wherein the adsorption liquid has a pH of 7.0.
The results indicate that when the pH of an adsorption liquid is adjusted to 6.6 or more and 7.4 or less, the IgG

adsorption percentage increases, whereby the IgG recovery percentage increases.

2. Experiment B

(1) Porous zirconia particles

EDTPA-bound porous zirconia particles (PCS140(SD)-P (0.0025M) as listed in Table 2 were used.

Table 2

| Test Ex. | Particle type | Absorption liquid | | Absorption percentage (%) | Desorption liquid | | Recovery percentage (%) |
|---|---|---|---|---|---|---|---|
| | | Concentration and component | pH | IgG | Concentration and component | pH | IgG |
| 11 | PCS140 (SD)-P (0.0025M) | 10mM PBS | 7. 0 | 72.8 | 100mM PBS | 6. 9 | 67. 3 |
| 12 | | 10mM PBS | 7.0 | 74. 1 | 100mM PBS<br>4mM EDTPA | 6. 6 | 70. 0 |
| 13 | | 10mM PBS | 7. 5 | 70. 8 | 100mM PBS | 7. 5 | 70. 8 |
| 14 | | 10mM PBS | 7. 5 | 83. 9 | 100mM PBS<br>4mM EDTPA | 7. 0 | 78. 1 |
| 15 | | 10mM PBS | 7.0 | 80.0 | 100mM PBS<br>2M NaCl | 7.0 | 61. 7 |
| 16 | | 10mM PBS | 7. 5 | 77. 1 | 100mM PBS<br>2M NaCl | 7. 5 | 33. 1 |
| 17 | | 10mM PBS | 7.0 | 95. 0 | 2M NaCl | 5.5 | 23.6 |

(2) Evaluation of adsorption and desorption

(2.1) Evaluation of adsorption

An adsorption liquid (500 μL) was added to a Spitz tube, and EDTPA-bound porous zirconia particles (PCS140(SD)-P (0.0025M)) (3 mg) were added to the liquid. After completion of sufficient dispersion of the EDTPA-bound porous zirconia particles, an IgG liquid (500 μg/500 μL) (500 μL) was added to the dispersion, and the resultant mixture was stirred overnight at 4°C under shielding from light.

The Spitz tube was subjected to centrifugation at 14,000 rpm for 5 minutes, to thereby precipitate the porous zirconia particles. The mass of non-immobilized IgG remaining in the supernatant was determined by means of SDS-PAGE (sodium dodecyl sulfate-polyacrylamide gel electrophoresis using Image Analysis Software CS Analyzer 4 (product of ATTO)). The difference between the initial IgG mass and the mass of non-adsorbed IgG was employed as the mass of adsorbed IgG. The ratio of the mass of adsorbed IgG to the initial IgG mass was calculated as an adsorption percentage (%).

(2.2) Evaluation of desorption

After the aforementioned evaluation of adsorption, desorption of IgG was evaluated by use of each desorption liquid listed in Table 2.

IgG was desorbed as follows. Specifically, after the aforementioned evaluation of adsorption, the supernatant was removed from the Spitz tube, and then each desorption liquid listed in Table 2 (500 μL) was added to the Spitz tube, followed by resuspension for desorption (elution) of IgG.

Subsequently, the Spitz tube was subjected to centrifugation at 14,000 rpm for 5 minutes, to thereby precipitate the porous zirconia particles. The mass of IgG desorbed from the porous zirconia particles and contained in the supernatant was determined by means of SDS-PAGE (sodium dodecyl sulfate-polyacrylamide gel electrophoresis using Image Analysis Software CS Analyzer 4 (product of ATTO)).

The thus-determined mass of IgG was employed as the mass of recovered IgG.

The ratio of the mass of recovered IgG to the initial IgG mass was calculated as a recovery percentage (%). In experiment C, a recovery percentage (%) was calculated in the same manner as described above.

(3) Test results

Test results are shown in Table 2.
The recovery percentage is higher in Test Examples 11 to 16 (wherein a neutral desorption liquid was used) than in Test Example 17 (wherein an acidic desorption liquid was used).

3. Experiment C

(1) Porous zirconia particles

Porous zirconia particle products as listed in Table 3 were used. In Table 3, "Rhinophase-AB" is a porous zirconia particle product of Zir Chrom Seperations Inc.

Table 3

| Test Ex. | Particle type | Absorption liquid | | Absorption percentage (%) | Desorption liquid | | Recovery percentage (%) |
|---|---|---|---|---|---|---|---|
| | | Concentration and component | pH | IgG | Concentration and component | pH | IgG |
| 18 | PCS140(SD) | 10mM PBS | 7. 0 | 69. 8 | 100mM PBS | 6. 9 | 69. 8 |
| 19 | Rhinophase-AB | 10mM PBS | 7. 0 | 26.5 | 100mM PBS | 6.9 | 14.6 |
| 20 | PCS140(SD) | 20mM MES<br>4M EDTPA<br>50mM NaCl | 2. 1 | 63.8 | 20mM MES<br>4M EDTPA<br>2M NaCl | 2.1 | 30.3 |
| 21 | Rhinophase-AB | | | 45. 5 | | | 6.5 |

(2) Evaluation of adsorption and desorption

(2.1) Test Examples 18 and 19

Evaluation of adsorption and desorption was performed in the same manner as employed in Test Example 11, except that porous zirconia particles as listed in Table 3 were used, and goat serum (500 μL) (IgG: 300 μg/500 μL) was used.

(2.2) Test Examples 20 and 21

Evaluation of adsorption and desorption was performed in the same manner as employed in Test Example 11, except that porous zirconia particles as listed in Table 3 were used, a solution containing 20mM MES (2-(N-morpholino)ethanesulfonic acid), 4M EDTPA, and 50mM NaCl was used as an adsorption liquid, and a solution containing 20mM MES, 4M EDTPA, and 2M NaCl was used as a desorption liquid. Test Examples 20 and 21 correspond to a Rhinophase-AB standard purification process which is a conventional technique.

(3) Test results

Test results are shown in Table 3.
Firstly, the results of Test Examples 18 and 20 are compared with each other. In Test Example 18, both the adsorption liquid and the desorption liquid are a neutral buffer. In Test Example 20, both the adsorption liquid and the desorption liquid are an acidic buffer. The adsorption percentage is 69.8% in Test Example 18 (wherein the adsorption liquid is neutral), whereas the adsorption percentage is 63.8% in Test Example 20 (wherein the adsorption liquid is acidic). Thus, there is no large difference in adsorption percentage between these Examples. However, the recovery percentage is 69.8% in Test Example 18 and 30.3% in Test Example 20; i.e., the recovery percentage in Test Example 18 is considerably higher than that in Test Example 20.

Next, the results of Test Examples 19 and 21 are compared with each other. In Test Example 19, both the adsorption liquid and the desorption liquid are a neutral buffer. In Test Example 21, both the adsorption liquid and the desorption liquid are an acidic buffer. The adsorption percentage is 26.5% in Test Example 19 and 45.5% in Test Example 21; i.e., the adsorption percentage in Test Example 21 is higher than that in Test Example 19. However, the recovery percentage is 14.6% in Test Example 19 and 6.5% in Test Example 21; i.e., the recovery percentage in Test Example 19 is considerably higher than that in Test

Example 21.

These results indicate that the use of a neutral buffer as a desorption liquid facilitates desorption of IgG adsorbed on the porous zirconia particles, resulting in an increase in recovery percentage.

In Test Example 18, the adsorption percentage is 69.8%, and the recovery percentage is 69.8%; i.e., IgG adsorbed on the porous zirconia particles is almost entirely desorbed.

4. Effects proven in Examples

The immunoglobulin purification method, which involves adsorption of an immunoglobulin onto porous zirconia particles in a neutral buffer and desorption of the immunoglobulin from the porous zirconia particles by means of a neutral desorption liquid, achieves a high immunoglobulin recovery percentage. In addition, the immunoglobulin purification method does not cause loss of the antibody nature of an immunoglobulin, since the method does not use an acidic solution or an alkaline solution.

<Other embodiments (variations)>

The invention is not limited to the aforementioned Examples and embodiments and may be carried out in various modes, so long as they do not deviate the scope of the invention.

(1) Although exemplary immunoglobulin purification device 1 and immunoglobulin production device 21 have been described above, the structure of the immunoglobulin purification device 1 or the immunoglobulin production device 21 may be appropriately modified depending on, for example, the type of an adsorption liquid or a desorption liquid, and the scale of the device.

(2) In the above description, the absorption/desorption unit 3 has a tubular portion. However, the tubular portion may be omitted.

Industrial Applicability

The immunoglobulin purification method of the present invention is suitable for use in purification of an immunoglobulin (antibody).

The immunoglobulin production method of the present invention is suitable for use in production of an immunoglobulin (antibody).

The immunoglobulin purification device of the present invention is suitable for use in purification of an immunoglobulin (antibody).

The immunoglobulin production device of the present invention is suitable for use in production of an immunoglobulin (antibody).

Description of Reference Numerals

1: immunoglobulin purification device
3: absorption/desorption unit
4: porous zirconia particles
5: adsorption liquid container
7: desorption liquid container
9: recovery container
11: switching unit
21: immunoglobulin production device
23: absorption/desorption unit

24: porous zirconia particles
25: adsorption liquid container
27: desorption liquid container
29: recovery container
31: switching unit

**Claims**

1. An immunoglobulin purification method **characterized by** comprising:

    an adsorption step of adsorbing an immunoglobulin onto porous zirconia particles in a neutral buffer; and
    a desorption step of desorbing the immunoglobulin adsorbed on the porous zirconia particles from the porous zirconia particles by means of a neutral desorption liquid.

2. An immunoglobulin purification method according to claim 1, wherein the desorption liquid is a phosphate buffer.

3. An immunoglobulin purification method according to claim 1 or 2, wherein the desorption liquid contains at least one species selected from the group consisting of a compound represented by the following formula (1):

    [F1]

    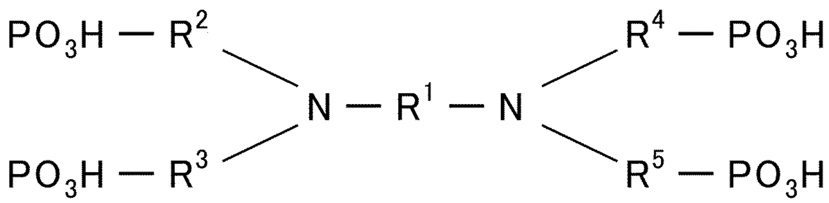
    (1)

    (wherein $R^1$ represents a C1 to C10 alkylene group; and each of $R^2$ to $R^5$, which may be identical to or different from one another, represents a C1 to C10 alkylene group) and a salt thereof.

4. An immunoglobulin purification device **characterized by** comprising:

    an adsorption unit for adsorbing an immunoglobulin onto porous zirconia particles in a neutral buffer; and
    a desorption unit for desorbing the immunoglobulin adsorbed on the porous zirconia particles from the porous zirconia particles by means of a neutral desorption liquid.

5. An immunoglobulin purification device according to claim 4, wherein the desorption liquid is a phosphate buffer.

6. An immunoglobulin purification device according to 4 or 5, wherein the desorption liquid contains at least one species selected from the group consisting of a compound represented by formula (1):

    [F2]

    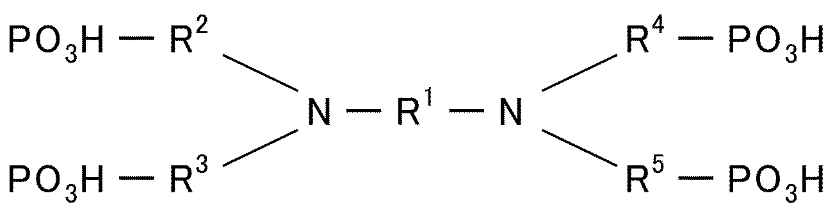
    (1)

    (wherein $R^1$ represents a C1 to C10 alkylene group; and each of $R^2$ to $R^5$, which may be identical to or different from one another, represents a C1 to C10 alkylene group) and a salt thereof.

7. An immunoglobulin production method **characterized by** comprising:

an adsorption step of adsorbing an immunoglobulin onto porous zirconia particles in a neutral buffer; and
a desorption step of desorbing the immunoglobulin adsorbed on the porous zirconia particles from the porous zirconia particles by means of a neutral desorption liquid.

**8.** An immunoglobulin production method according to claim 7, wherein the desorption liquid is a phosphate buffer.

**9.** An immunoglobulin production method according to claim 7 or 8, wherein the desorption liquid contains at least one species selected from the group consisting of a compound represented by formula (1):

[F3]

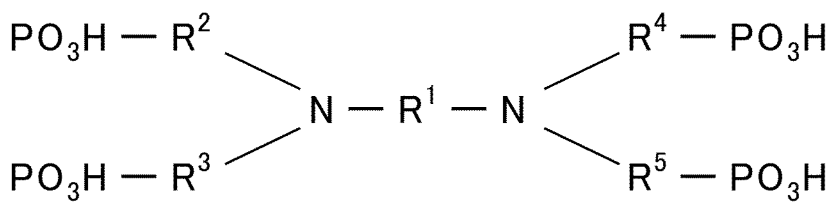

(1)

(wherein $R^1$ represents a C1 to C10 alkylene group; and each of $R^2$ to $R^5$, which may be identical to or different from one another, represents a C1 to C10 alkylene group) and a salt thereof.

**10.** An immunoglobulin production device **characterized by** comprising:

an adsorption unit for adsorbing an immunoglobulin onto porous zirconia particles in a neutral buffer; and
a desorption unit for desorbing the immunoglobulin adsorbed on the porous zirconia particles from the porous zirconia particles by means of a neutral desorption liquid.

**11.** An immunoglobulin production device according to claim 10, wherein the desorption liquid is a phosphate buffer.

**12.** An immunoglobulin production device according to claim 10 or 11, wherein the desorption liquid contains at least one species selected from the group consisting of a compound represented by formula (1):

[F4]

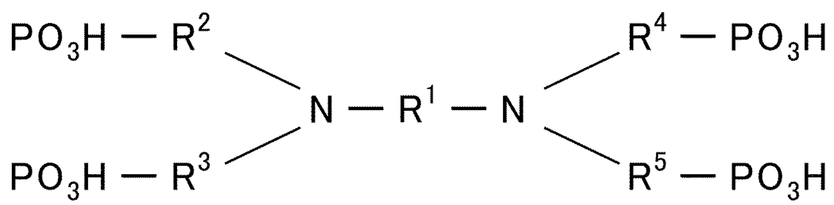

(1)

(wherein $R^1$ represents a C1 to C10 alkylene group; and each of $R^2$ to $R^5$, which may be identical to or different from one another, represents a C1 to C10 alkylene group) and a salt thereof.

# Fig. 1

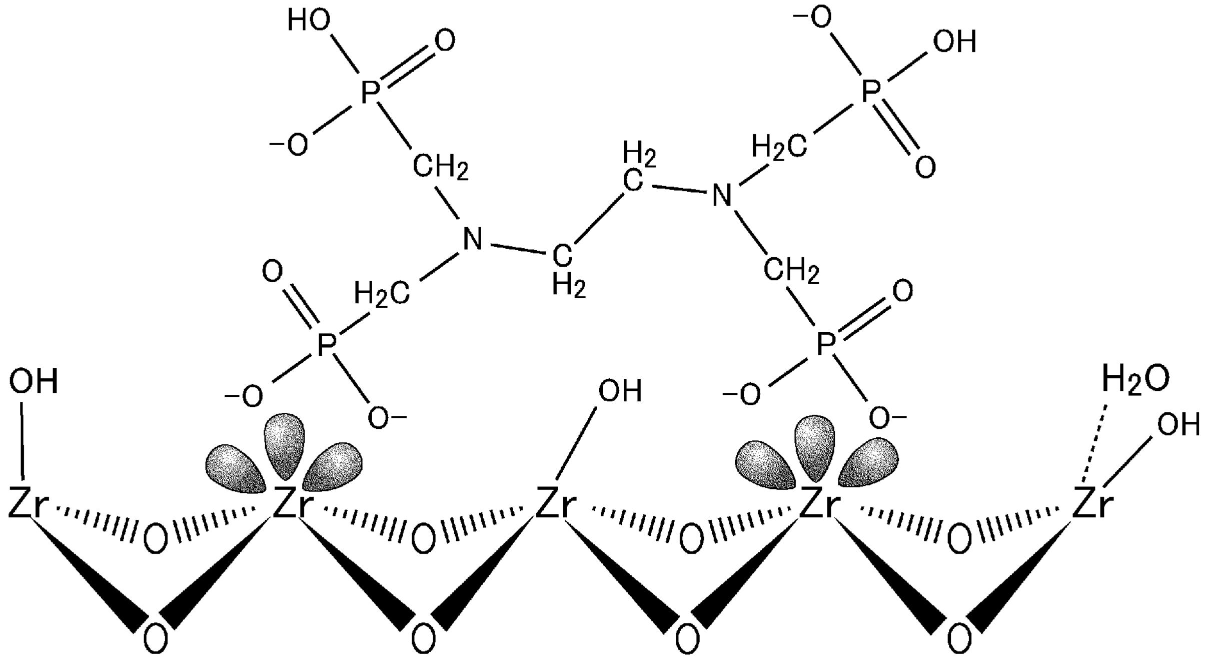

Fig. 2

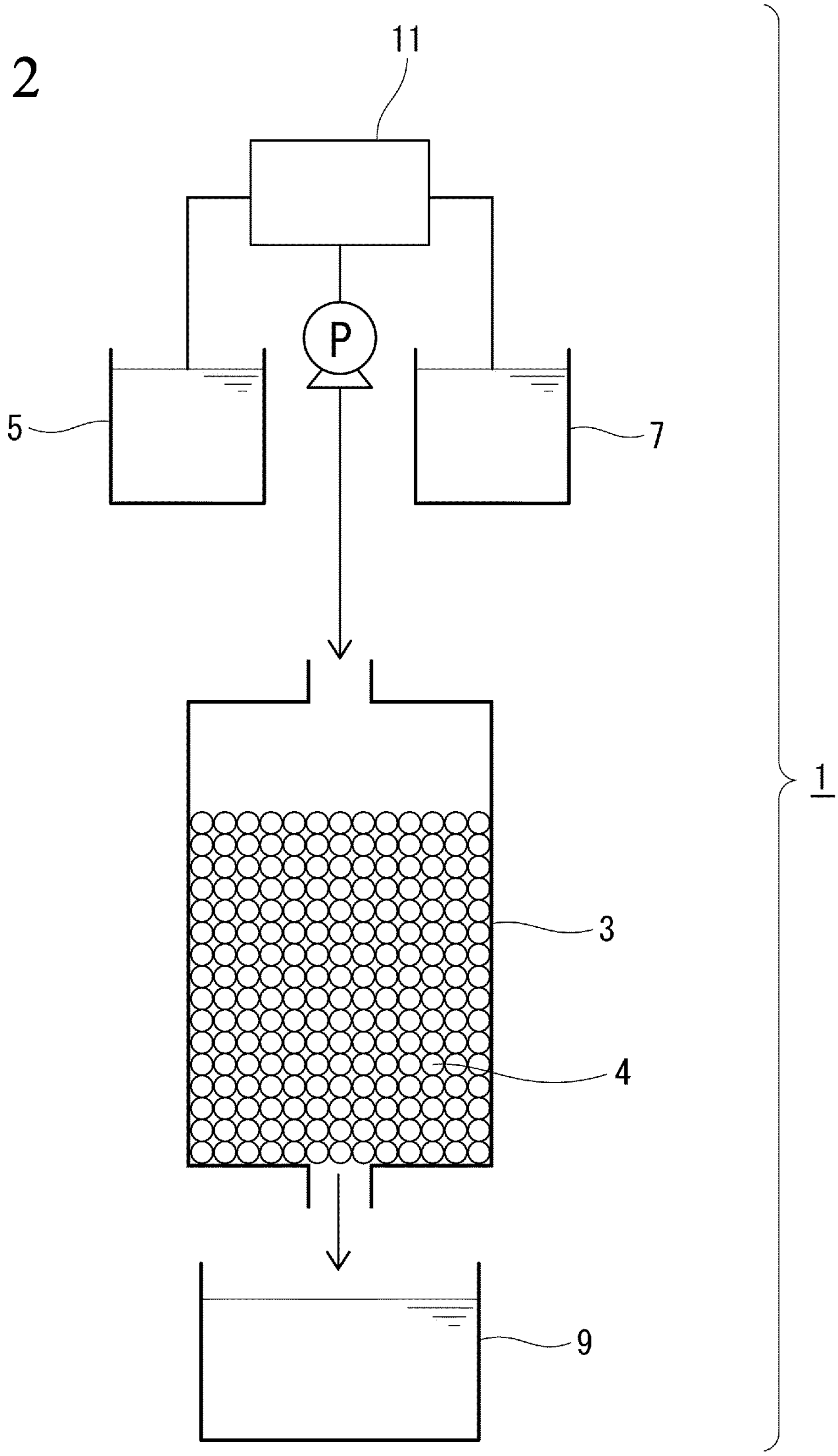

Fig. 3

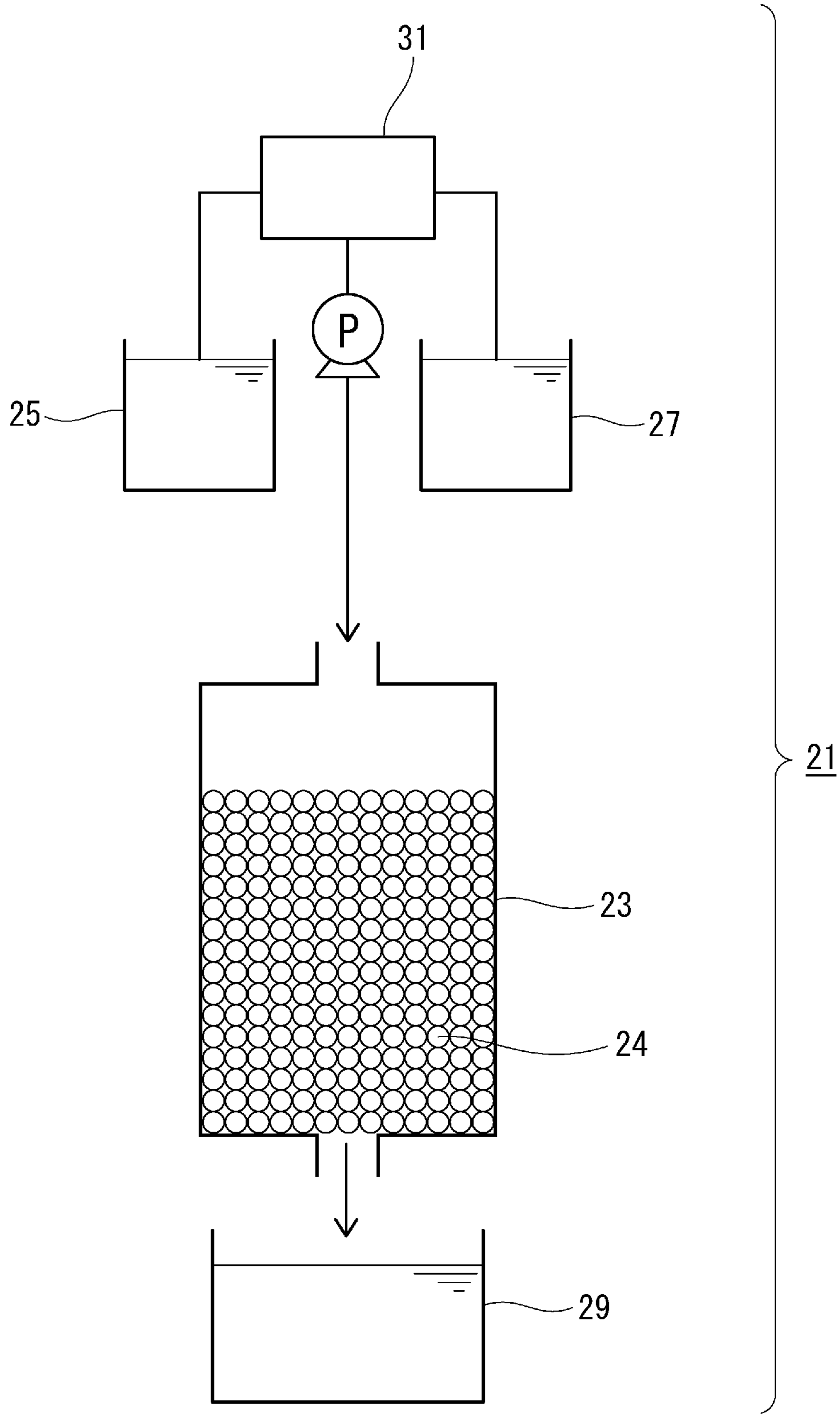

## INTERNATIONAL SEARCH REPORT

International application No.
PCT/JP2020/001499

A. CLASSIFICATION OF SUBJECT MATTER
C07K 1/22(2006.01)i; C07K 16/00(2006.01)i; C12M 1/00(2006.01)i; B01J 20/06(2006.01)i
FI: C07K1/22; C12M1/00; B01J20/06 A; C07K16/00
According to International Patent Classification (IPC) or to both national classification and IPC

B. FIELDS SEARCHED
Minimum documentation searched (classification system followed by classification symbols)
C07K1/22; C07K16/00; C12M1/00; B01J20/06

Documentation searched other than minimum documentation to the extent that such documents are included in the fields searched

| | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)
WPI; JSTPlus/JMEDPlus/JST7580 (JDreamIII);
CAplus/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| A | US 2004/0007530 A1 (MCNEFF, Clayton V.) 15.01.2004 (2004-01-15) paragraphs [0123]-[0131] | 1-12 |
| A | CLAUSEN, A. M. et al., "Purificatlon of monoclonal antibodies from cell culture supernatants using a modified zirconia based cation-exchange support", Journal of Chromatography A, 1999, vol. 831, issue 1, pp. 63-72 in particular, Methods | 1-12 |
| A | MOWRY, M. C., et al., "Production and purification of a chimeric monoclonal antibody against botulinum neurotoxin serotype A", Protein Expression and Purification, 2004, vol. 37, issue 2, pp. 399-408 in particular, Materials and methods | 1-12 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 March 2020 (23.03.2020) | 31 March 2020 (31.03.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2020/001499

C (Continuation). DOCUMENTS CONSIDERED TO BE RELEVANT

| Category* | Citation of document, with indication, where appropriate, of the relevant passages | Relevant to claim No. |
|---|---|---|
| A | SUBRAMANIAN, A., et al., "Use of spray-dried zirconia m1crospheres in the separation of immunoglobulins from cell culture supernatant", Journal of Chromatography A, 2000, vol. 890, issue 1, pp. 15-23 in particular, Methods | 1-12 |
| A | JP 2017-047365 A (NATIONAL INSTITUTE OF ADVANCED INDUSTRIAL SCIENCE AND TECHNOLOGY) 09.03.2017 (2017-03-09) claims, examples | 1-12 |

Form PCT/ISA/210 (continuation of second sheet) (January 2015)

**INTERNATIONAL SEARCH REPORT**
Information on patent family members

International application No.
PCT/JP2020/001499

| Patent Documents referred in the Report | Publication Date | Patent Family | Publication Date |
|---|---|---|---|
| US 2004/0007530 A1 | 15 Jan. 2004 | (Family: none) | |
| JP 2017-047365 A | 09 Mar. 2017 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2017047365 A